# EUROPEAN PATENT APPLICATION

(11) **EP 1 378 262 A2**
(43) Date of publication of application: **07.01.2004**
(21) Application number: 03077843.5
(22) Date of filing: 06.04.1999
(51) Int. Cl.: A61M 25/01

(54) **Modified guidewire for left ventricular access lead**

(30) Priority: 12.06.1998 US 97101; 01.10.1998 US 164891
(62) Divisional of application: 99916410.6
(71) Applicant: CARDIAC PACEMAKERS, INC., St. Paul, Minnesota 55112-5798 (US)
(72) Inventor: Peterfeso, Randall M., St. Paul Minnesota 55101 (US); Hinders, Mary N., Murrieta California 92562 (US); Tockman, Bruce A., Scandia Minnesota 55072 (US); Greenland, John S., San Diego California 92128 (US); Westlund, Randy W., Minneapolis Minnesota 55407 (US)
(74) Representative: Charig, Raymond Julian

(57) **Abstract**

An improved guide wire (20) for assisting in implantation of a cardiac lead includes three sections (22, 24, 26). The most distal zone (22) is sufficiently floppy to prevent trauma to the vessel walls through which the guide wire (20), and lead are inserted, An intermediate zone (24) is generally stiffer, and has a cross section less than or equal to the cross section of the distal zone (22). The third zone (26) is stiffer yet, and is joined to the intermediate zone by a shoulder (34) to transfer force to the lead.

## Description

### CROSS REFERENCE TO THE RELATED APPLICATION

This patent application is a continuation-in-part of copending application Serial No. 09/097,101, filed June 12, 1998.

### BACKGROUND OF THE INVENTION

### I. Field of the Invention

The invention relates to the implantation and placement of cardiac leads used in combination with cardiac rhythm management devices, e.g., heart pacemakers or defibrillators, to monitor and control the rhythm of the heart. This invention is more particularly directed toward a guidewire configuration adapted to assist in the implantation and placement of cardiac leads having electrodes that are to reside in the distal branches of the coronary venous system, the great cardiac vein, or coronary sinus.

### II. Discussion of the Prior Art

Placement of cardiac leads in the distal branches of the coronary venous system, the great cardiac vein, or the coronary sinus is a difficult task. Often when deploying the lead there comes a point at which the lead cannot be advanced further into the vascular system using standard techniques and equipment. All too often this point is not the optimal position for the lead's electrode either sensing cardiac activity or delivering therapy to the heart.

There are several reasons which make proper placement of the lead difficult. These include (1) friction between the vasculature and the lead; (2) partial obstruction of the vasculature; (3) unusually shaped bifurcations in the vasculature; and (4) accumulative friction between lead, guide catheter and guidewire. Prior efforts to resolve such problems included the use of a stiffer guidewire. While stiffer guidewires offer additional support, they may impede advancement due to their relative size with respect to the lumen of the lead.

Additionally, when proper placement of the lead is achieved, problems arise during guide catheter extraction. All too often, the act of extracting the guide catheter causes the lead to be dislodged from the implanted position. Standard guidewires and stylets are not suitable for maintaining position while the guide catheter is removed due to insufficient stiffness, lack of appropriate force transmission features, and friction between the guidewire and lumen wall of the coronary vein lead.

The present invention is deemed to be an improvement over such prior art guidewires. It is more effective in properly placing the lead. It is also less likely to cause a properly placed lead to become dislodged during extraction of the guide catheter and the guidewire itself.

### SUMMARY OF THE INVENTION

In accordance with the present invention, there is provided a guidewire comprised of at least three zones. Each zone differs from the other two in stiffness and flexibility. Each zone also has geometric characteristics which assist in proper placement of the lead and further assist in preventing dislodgement of the lead as the guide catheter is extracted and as the guidewire itself is extracted.

Specifically, the first and most distal zone is intended to be sufficiently floppy to prevent trauma to the surrounding vessel walls when the guidewire is used for deploying a coronary vein lead. This zone includes a spiral wound portion surrounding a thin, solid ribbon core and a spherical tip. The second intermediate zone, is generally stiffer and comprises a solid wire or spiral wound wire having a cross-sectional diameter that does not exceed the cross-sectional diameter of the first zone. The third most proximal zone is of a larger diameter and is stiffer than the first and second zones. The third zone comprises a wire or tube which is manipulated to apply advancement forces during deployment of the lead and counter forces during extraction of the guide catheter. The diametric transition between the second and third zones is abrupt and ideally corresponds to a matching feature in the lead so that this transition is the point where most of the advancement forces and counter forces are transmitted to the lead.

Other improvements also exist. For example, the improved guidewire (or portions thereof) can be provided with a hydrophilic coating to produce a highly lubricious surface. The presence of such a surface reduces friction between the lumen wall of the lead and the guidewire thereby reducing the risk that the lead will be dislodged during extraction of the guidewire. Likewise, a locking mechanism can be provided between the lumen of the lead and the guidewire so that forces applied to the guidewire are transmitted to the lead through the locking mechanism.

### DESCRIPTION OF THE DRAWINGS

The foregoing features, objects and advantages of the present invention will become more clear to those skilled in the art from the following detailed description of a preferred embodiment, particularly when considered in conjunction with the accompanying drawings in which like numerals in the several views refer to corresponding parts.

Figure 1 is a view showing an intravenous cardiac lead having an electrode positioned in a coronary vein.

Figure 2 is a perspective view of a preferred embodiment of the guidewire of the present invention.

Figure 3 is a cross-sectional view of a cardiac lead with a guidewire positioned within the lumen of the lead.

Figure 4 is a plan view of an alternative embodiment of a guidewire made in accordance with the present invention.

Figure 5 is a cross-sectional view of a cardiac lead with a guidewire of the type shown in Figure 4 positioned within the lumen of the lead.

### DETAILED DESCRIPTION OF THE INVENTION

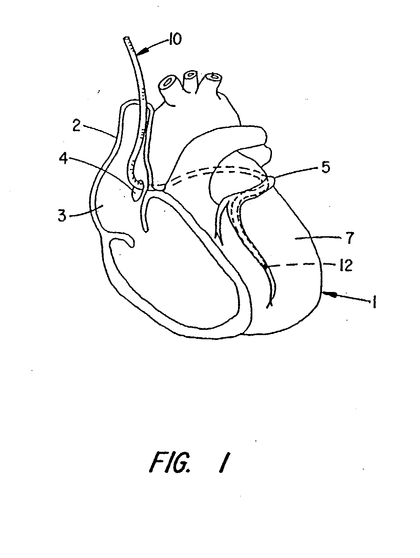

Figure 1 shows a human heart 1 with a coronary lead 10 passing through the superior vena cava 2, the right atrium 3, and the coronary sinus 4 into the great vein of the heart 5 so that an electrode 12 on the lead 10 is properly positioned in a branch of the coronary vein. When positioned as shown, the electrode 12 can be used to sense the electrical activity of the heart or apply stimulating pulses to the left ventricle 7 without the electrode being positioned within the left ventricular chamber. The present invention is directed toward elongated wires useful in placing the electrode in the vasculature. Such wires are generally referred to as "guidewires". As used herein, the term "guidewire" includes both wires used to position the electrode and wires used to retain the electrode in the proper position as a guide catheter is removed.

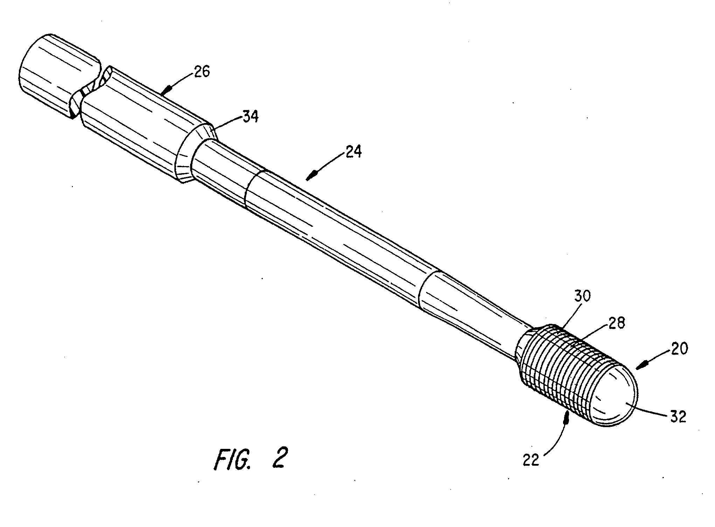

Figure 2 shows a first preferred embodiment of a guidewire 20 advantageously used to position the coronary lead 10 as shown in Figure 1 and retain the coronary lead in that position as a guide catheter is removed. Guidewire 20 has three zones, a Distal zone 22, intermediate zone 24, and a proximal zone 26.

The distal zone 22 preferably is 1-1.5 inches long. The distal zone 22 is circular in cross-section and has a cross-sectional diameter of approximately 0.014 inches. The distal zone 22 comprises an internal shapeable ribbon core member 28, a spiral winding 30 and a spherical tip 32. The internal ribbon core member 28, as it extends distally, tapers from about 0.005 inches to about 0.001 inches. This construction is sufficiently floppy such that there is no trauma induced by the guidewire to a surrounding vessel wall. This construction also allows it to be capable of being biased so as to aid in steering through the vasculature.

The intermediate zone 24 is generally stiffer than the distal zone 22. The intermediate zone comprises a solid wire having a circular cross-section. The cross-sectional diameter of the wire can vary depending upon the performance needs, but should not exceed the cross-sectional diameter of distal zone 22. The length of intermediate zone 24 can also vary, but preferably will be one to four inches long.

The proximal zone 26 is made of a wire or tubing and is the stiffest and longest section of the guidewire 20. The proximal zone 26, being the stiffest and most proximal, is the portion handled and used to apply forces during deployment and guide catheter extraction. Preferably the overall length of the guidewire 20 will be in the range of five feet. The cross-sectional diameter of the proximal zone is larger than the cross-sectional diameter of the distal zone 22 and the intermediate zone 24. For example, if the distal and intermediate zones have a diameter of approximately 0.014 inches, the proximal zone will have a diameter of approximately 0.022 inches. The diametrical transition between the proximal and intermediate zones taper, though abruptly, from 0.022 inches to 0.014 inches. As discussed below, this diametrical transition constitutes a shoulder 34 through which most of the advancement and counter forces are transmitted between the lead 10 and guidewire 20.

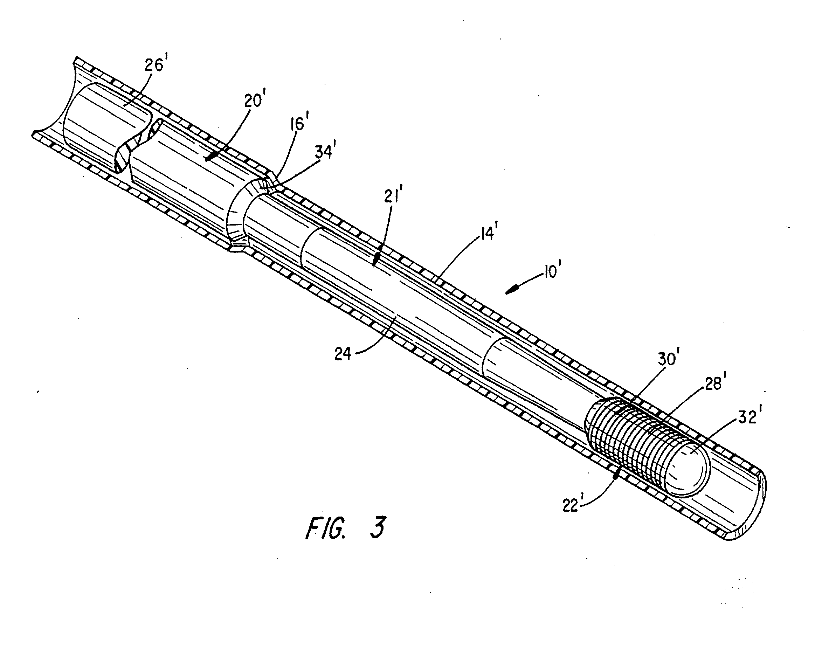

Figure 3 shows the guidewire 20 of Figure 2 positioned within a lumen 14 of the coronary lead 10. The lumen 14 has a projection 16 which corresponds to the shoulder 34 of the guidewire 20. When the shoulder 34 engages the projection 16, advancement forces applied to the guidewire 20 are transferred to the lead 10 through the shoulder 34 and projection 16 or reduction in lumen diameter.

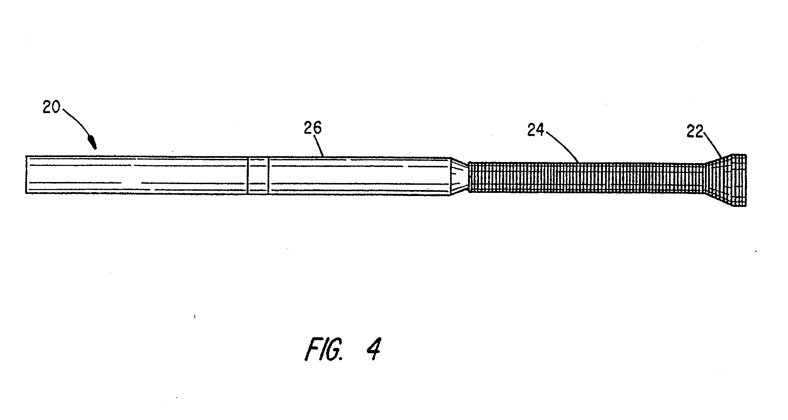

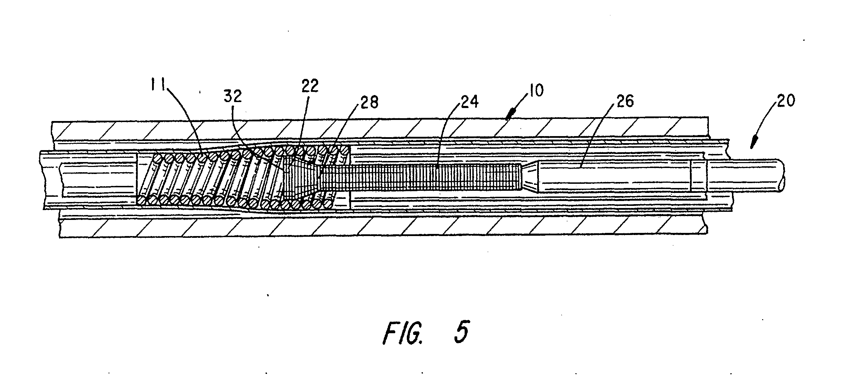

Figures 4 and 5 show a second preferred embodiment of a guidewire 20, specifically referred to as a removal wire. This embodiment is ideally suited for use during removal of a guide catheter. As such, it may be used to replace a standard guidewire after insertion of the lead but before removal of the guide catheter. Again, the guidewire 20 of this embodiment has three zones -- a distal zone 22, an intermediate zone 24, and a proximal zone 26. The guidewire 20 shown in Figure 4 is dimensioned in a fashion similar to the guidewire shown in the other three figures. The primary difference between the removal wire shown in Figure 4 and the guidewire shown in the other figures is that the tip 32 is not attached to the core 28 by a solder joint. Also, the tip 32 of this embodiment is not intended to exit the distal end of the lead 10, thus it is not shapeable or steerable in the vasculature. It may be used to lock into the lead 10 and transmit force to the lead tip, but only as used in conjunction with the lead. The tip 32 of this embodiment is not considered atraumatic whereas the tip in the embodiment shown in Figure 2 is designed to be extremely atraumatic. This spiral wound wire is secured to the proximal zone 26 by a solder joint. The direction of the winding will preferably be opposite that of any winding 11 of the lead 10 itself. This allows for better tracking through the central lumen of the lead. The distal zone 22 is a continuation of the spiral winding of the intermediate zone. However, the diameter of the winding is increased to form the distal zone. The distal tip 32 of the coil which forms the intermediate end distal zones is cut square and not attached to a core wire or the like. Thus, as the guide catheter is withdrawn, the square cut of tip 32 seats in the taper of the coil 11 of the lead preventing the lead from withdrawing as the guide catheter is pulled free. Furthermore, as the removal wire is pulled free from the lead, the square, unattached coil tip 32 slightly distends and easily frees itself from the tapered coil section of the lead. This feature allows for easy, predictable removal of the removal wire from the lead, thus preventing loss of lead position upon its withdrawal.

In either embodiment, a hydrophilic coating can be applied to create a surface 21 on the guidewire 20 that is highly lubricious. Alternatively, all surfaces of guidewire 20 can be coated with a hydrophilic coating or polytetrafluoroethylene (PTFE), or some other dry lubricious material, i.e. silicone film. This serves to reduce friction between the guidewire 20 and lead lumen 14 thereby reducing the risk that the lead 10 will be dislodged from its proper position as the guidewire 20 is extracted.

## Claims

1. An intravenous lead insertion assembly, comprising
a catheter having a proximal end and a distal end;
an intravenous lead (10) at least partially located within the catheter and having at least one electrode (12) and a lumen (14) extending from a proximal end of the lead to a distal end of the lead, the lumen having a first opening through the proximal end of the lead and a second opening through the distal end of the lead;
a guidewire for facilitating advancement of the intravenous lead (10) into the coronary sinus (4), said guidewire sized to extend through the first and second openings in said lumen and past the distal end of the lead during implantation of the lead, and being removable from the lead after placement of the lead in the coronary sinus; and
a removal wire (20) sized to extend through the first opening and into the lumen (14) of the intravenous lead (10), the removal wire having a distal end portion (22) including a lead contacting portion (32) for contacting the lead to facilitate securing the lead during a removal of the catheter from around the lead.

2. The intravenous lead insertion assembly according to claim 1, wherein the lumen (14) of the intravenous lead (10) is of a size to separately receive the guidewire and the removal wire (20), but is not of a size to receive the guidewire and the removal wire simultaneously.

3. The intravenous lead insertion assembly according to claim 1, wherein the distal end of the intravenous lead (10) extends distal of the distal end of the catheter prior to removal of the catheter.

4. The intravenous lead insertion assembly according to claim 1, wherein the contacting portion of the removal wire (20) abuts a portion (11) of the intravenous lead.

5. The intravenous lead insertion assembly according to claim 1, wherein the contacting portion (32) of the removal wire (20) contacts the lumen (14) of the intravenous lead.

6. The intravenous lead insertion assembly according to claim 1, wherein the contacting portion (32) of the removal wire (20) includes a transition portion located between a larger diameter section (22) of the removal wire and a smaller diameter section (24) of the removal wire.

7. The intravenous lead insertion assembly according to claim 6, wherein the lumen (14) of the intravenous lead (10) includes a first diameter portion and a second diameter portion, the second diameter portion being smaller than the first diameter and located distal of the first diameter portion.

8. The intravenous lead insertion assembly according to claim 7, wherein the lumen (14) of the intravenous lead (10) includes a tapered portion (16) between the first and second diameter portions.

9. The intravenous lead insertion assembly according to claim 1, wherein the contacting portion (32) of the removal wire provides a frictional engagement with the lumen (14) of the intravenous lead (10).

10. The intravenous lead insertion assembly according to claim 1, wherein the removal wire (20) includes a coiled member (32) forming a squared distal tip.

11. The intravenous lead insertion assembly according to claim 1, wherein the lumen (14) of the intravenous lead (10) is formed by a helical coil (11).

12. The intravenous lead insertion assembly according to claim 11, wherein the contacting portion (32) of the removal wire (20) engages the helical coil (11) of the lead.

13. The intravenous lead insertion assembly of any preceding claim in which the intravenous lead includes at least one pacing electrode.

14. The intravenous lead insertion assembly of any one of claims 1 to 12 in which the intravenous lead includes at least one defibrillation electrode.
